(19) Europäisches Patentamt
European Patent Office
Office européen des brevets

(11) EP 0 684 037 B1

(12) EUROPEAN PATENT SPECIFICATION

(45) Date of publication and mention of the grant of the patent:
**14.11.2001 Bulletin 2001/46**

(51) Int Cl.[7]: **A61K 7/32**

(21) Application number: **95301807.4**

(22) Date of filing: **17.03.1995**

(54) **Deodorant compositions**

Desodorisierende Präparate

Compositions déodorantes

(84) Designated Contracting States:
**CH DE ES FR GB IT LI NL SE**

(30) Priority: **31.03.1994 GB 9406574**

(43) Date of publication of application:
**29.11.1995 Bulletin 1995/48**

(73) Proprietors:
- **UNILEVER PLC**
**London EC4P 4BQ (GB)**
Designated Contracting States:
**GB**
- **UNILEVER N.V.**
**3013 AL Rotterdam (NL)**
Designated Contracting States:
**CH DE ES FR IT LI NL SE**

(72) Inventor: **Wight, Gordon R., Unilever Research**
**Wirral, Merseyside, L63 3JW (GB)**

(74) Representative:
**Rots, Maria Johanna Francisca et al**
**Unilever PLC,**
**Patent Division,**
**Colworth House**
**Sharnbrook, Bedford MK44 1LQ (GB)**

(56) References cited:
**EP-A- 0 519 531** **WO-A-93/01793**
**FR-A- 2 380 341**

Remarks:
The file contains technical information submitted after the application was filed and not included in this specification

EP 0 684 037 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

## Description

This invention relates to deodorant compositions. In particular, it relates to deodorant compositions which comprise certain combinations of fragrance compositions, in conjunction with a specific class of deodorant active materials. The combined composition is particular suitable for use as or in personal body deodorant compositions, such as underarm deodorant products.

Combinations of fragrance compositions which exhibit a deodorancy effect in their own right are know. For example, EP 0 003 171 and EP 0 003 172 (Unilever), and US 4 304 679 (Lever) (and the references cited therein) give extensive known examples of perfumery compositions, especially with reference to their use in detergents.

EP 0 003 171 and EP 003 172 also disclose compositions in which the perfume compositions have a deodorant effect when used.

Further examples of perfumery compositions, as well as their application to enzyme-containing detergents, are given in JP 57-85898 and JP 57-85900 (Lion Corp), and in USP 4 515 705 (Procter & Gamble).

EP 0 147 191 (Unilever) gives further examples of perfumery compositions, having not only deodorant effect but also stability in the presence of bleaching compositions.

It is also proposed in GB 1 589 866 to incorporate 0.3-3% of ester of citrate or acetylcitrate (e.g. triethyl citrate) as a deodorant in a soap bar.

In addition, EP 404 470 (Quest International) discloses certain combinations of fragrance materials, which in themselves have a relatively low Odour Intensity Index, but a relatively high Malodour Reduction Value (as defined therein). Such compositions are described as being suitable for use in detergent compositions soaps, fabric conditioning compositions, or personal body deodorant compositions.

FR 7 804 124 (Unilever) discloses fragrance materials which have a relatively high Malodour Reduction Value (as defined therein) in deodorant products.

GB 2013493 (Unilever) also describes the possibility of incorporating perfume compositions which have a deodorant effect into skin treatment products, such as talcs, creams and lotions.

It is also generally known that certain inorganic salts, for example those of aluminium magnesium, lanthanum, and zinc, can be used in cosmetic deodorant compositions. Such salts convey to the composition a relatively high deodorant effect, and the resulting compositions may be suitable for topical application to the human skin.

We have surprisingly found that certain combinations of fragrance materials, in combination with certain classes of deodorant active metal salts, can be used in cosmetic deodorant compositions which are suitable for topical application to the human skin, and which demonstrate a remarkably high efficacy. Such a deodorant effect may be greater than would be obtained by using the combination of fragrance materials or the deodorant active materials on their own in cosmetic compositions; the combination thereof may also demonstrate synergistic efficacy.

In its broadest sense, the invention thus comprises a deodorant composition for topical application to the human skin, comprising a composition of non-encapsulated fragrance materials which have an Odour Intensity Index of less than about 110, when tested according to an Odour Intensity Test as described herein, and a Deodorant Value greater than 0.25, as measured by the Deodorant Value Test as described herein, and a deodorant active metal salt or complex in a cosmetically suitable vehicle.

Preferably, the composition of fragrance materials used in the composition according to the invention has a Deodorant Value of greater than 0.5; more preferably the Deodorant Value is between 0.50 and 3.5, even more preferably between 0.9 and 3.5 as measured by the Deodorant Value Test.

Preferably, the inorganic metal salt or complex in the composition comprises a zinc salt or complex, and most preferably comprises zinc phenol sulphonate. Aluminium or zirconium containing deodorant active salts or complexes may also be preferred.

According to one preferred aspect of the invention, the combination of fragrance materials concerned can confer deodorant effects in use, even though they themselves may have a low or even imperceptible level of fragrance (i.e. a low odour intensity). This may be of particular advantage where an intense fragrance is not required, but the deodorancy effect therefrom is desired.

Thus, according to one aspect of the invention, the deodorant composition for topical application to the human skin comprises a composition of non-encapsulated fragrance materials having

a) an Odour Intensity Index of less than about 110, (often less than about 105, and preferably less than about 100), when tested according to an Odour Intensity Test as described below, and

b) a Malodour Reduction Value of at least about 0.25, preferably, at least about 0.5, when tested according to the test procedure set out in European Patent Application No. 0. 147 191 or an Odour Reduction Value of about 0.25, preferably at least about 0.5, when tested according to the test procedure set out in European Patent Application No. 0 003 172, both of which specifications are hereby incorporated herein by reference but preferably a Deodorant

Value of 0.25, preferably at least 0.5, as measured by the Deodorant Value Test described herein, and an effective amount of an inorganic deodorant active material.

It is understood that in this specification, expressions such as 'perfume' and 'fragrance' extend to compositions of which the odour intensity may be so low as to be imperceptible in use.
A suitable selection of examples of perfumery materials for incorporation into such compositions is for example provided in the examples below. More generally, any of a wide range of perfumery materials may be incorporated into the compositions, provided that the basis of selection is such as to provide a deodorant effect, and the odour intensity index of the resulting composition is as defined above.
Extensive directions for the selection of materials in order to provide a deodorant effect are given for example in EP 0 147 191, EP 0 003 172, and USP 4 304 679, all three of which are incorporated herein by reference.
In this embodiment it is helpful if the bulk of the individual ingredients chosen for the composition also individually possess an Odour Intensity Index less than about 110, preferably less than about 100, or even lower. Small quantities of more intense materials may however be tolerated, e.g. for the purpose of adjusting the mild perfume note which may be given by the overall composition.
In a number of particular embodiments, the fragrance compositions can comprise at least 30% by weight of a musk, e.g. at least 35% or at least 40%. Where musks are present, either in these or in other amounts, they can usefully be selected from musks such as galaxolide (TM) (IFF) (in class 6 defined below) and or Traseolide (TM) (Quest).
According to a further aspect of the invention, there is provided a deodorant agent for topical application to the human skin, comprising perfume ingredients containing

a) optionally at least about 0.15% of a musk, based on the weight of the deodorant composition.

b) at least about 0.35% of other perfume components, provided that the fragrance components (a) and (b) together have an Odour Intensity Index of less than 100.

and provided that the aggregate of fragrance components together pass any of the odour reduction tests cited above, in combination with an effective amount of an inorganic deodorant active material.
The combinations of fragrance materials maybe used in compositions according to the invention at levels of 0.01-5%, more preferably 0.1-3%, even more preferably 0.3-2% by weight of the composition.
Preferably the deodorant active metal salt is present in compositions according to the invention at a level of 0.1-12%, more preferably 2-7% by weight of the composition.
In certain embodiments, the invention comprises a deodorant perfume which comprises deodorant perfume components, preferably having a Deodorant Value of from 0.25 to 3.5, and also having the characteristic low odour intensity as defined herein.
Such deodorant perfumes can comprise from 50 to 100% by weight of deodorant perfume components and from 0 to 50% by weight of further ingredients, the deodorant perfume components for example preferably having a Lipoxidase-Inhibiting Capacity of at least 50% or a Raoult Variance Ratio (otherwise known as the morpholine test) of at least 1.1. The components of the deodorant perfume maybe allocated to one of six classes consisting of:

Class 1: Phenolic substances
Class 2: Essential oils, extracts, resins and synthetic oils (denoted "AB")
Class 3: Aldehydes and ketones
Class 4: Nitrogen-containing compounds and polycyclic compounds
Class 5: Esters
Class 6: Alcohols and ethers

provided that where a deodorant perfume component can be assigned to more than one class, it is allocated to the class having the lower or lowest number:
Said components being so selected that:

a) the deodorant perfume contains at least five different components, preferably at least one from each of class 1, 2 and 4;

b) the deodorant perfume contains components from at least four of the six classes; and

c) any component present in the deodorant perfume at a concentration of less than 0.5% by weight of the said perfume is eliminated from the requirements of (a) and (b),

said deodorant perfume having a Malodour Reduction Value of from 0.25 to 3.5.

Product Form

The composition according to the invention can take the form of liquid or solid products, each of which is suited to or adapted for topical application to the human skin. One convenient form of the product is as a solid stick, usually contained in a suitable holder to enable it to be applied to the area of the skin, particularly the underarm, where it is required.

A preferred form of the composition of the invention is a lotion or cream suitable for dispensing from a roll-on dispenser fitted with a ball valve, to enable the product to be rolled onto or applied to the skin in a conventional fashion. A further example of the composition of the invention is as a liquid composition suitable for dispensing with a finger operated pump spray or hand operated squeeze spray, to deliver to the skin a finely divided liquid spray, without the use of propellant gases.

Alternatively, the composition according to the invention may be delivered as an aerosol, using a conventional pressurised apparatus with a propellant gas.

Cosmetic Adiuncts

The composition may additionally comprise other components typically found in deodorant compositions for topical application to the skin, including;

- linear or cyclic volatile silicones, including polydimethyl siloxanes such as Dow Corning 344 Fluid and Dow Corning 345 Fluid, and volatile hexamethyldisiloxane such as Dow Corning 200 Fluid,
- non-volatile silicones, including polydimethylsiloxane such as Dow Corning Fluids,
- thickeners, such as clays, for example Bentone 38, and silicas, for example Aerosil 200,
- skin feel improvers, such as talc and finely divided polyethylene, for example Acumist B18,
- cosmetically acceptable vehicles, such as water, anhydrous ethanol, aqueous ethanol, glycols such as propylene glycol or butylene glycol, and emollients,
- gelling agents, such as stearyl alcohol, sodium stearate or waxes, such as castor wax,
- perfumes,
- preservatives, and
- other cosmetically acceptable adjuncts conventionally employed in stick, roll-on, propellant driven and propellant-free spray deodorant products.

The Deodorant Value Test

In this test the deodorant value of a deodorant composition is measured by assessing its effectiveness when applied directly to the skin. The composition is applied to the axillae (armpits) of a panel of human subjects, and its effect at reducing malodour is subsequently assessed.

The test uses a team of 3 or 4 experienced female under-arm odour assessors. These are selected on the basis that they are able to rank correctly the odour intensities of a series of isovaleric acid solutions listed below. The intensities of this series of solutions forms the basis of the 0-5 category scale used to quantify under-arm odour subjectively in the test.

Malodour Category Scale

| SCORE | ODOUR LEVEL | AQUEOUS ISOVALERIC ACID CONC, ml/litre |
|---|---|---|
| 0 | no odour | 0 |
| 1 | slight | 0.013 |
| 2 | definite | 0.053 |
| 3 | moderate | 0.220 |
| 4 | strong | 0.870 |
| 5 | very strong | 3.57 |

New assessors are trained by participation alongside experienced assessors in the tests of deodorancy of underarm products. Training is complete when the new assessor is able to quantify underarm odour in the present protocol on the same basis as experienced assessors. Discrimination between underarm odour and residual product perfume in the total axillary odour is a key part of the skill of an expert odour assessor.

The panel consists of up to 50 subjects who have been selected by the expert odour assessors on the basis that their body odour is not unusually weak or strong, or uneven between axillae. The panellists are denied the use of any underarm products, and are provided with a non-deodorant soap bar for home use.

The test is run over a five day period, and includes 4 product application-odour assessment cycles. The period between product application to subjects and the assessment of under-arm odour is 5 hours and 24 hours. Only panellists who have not participated in any testing of under-arm products for at least a week previously can take part in this test, because product carry-over effects can occur.

On the first day of the test, the panellist's axillae are washed by the assessors using unperfumed soap. A standard technique is used in which a wet flannel is soaped for 15 seconds, the axilla washed with the flannel for 30 seconds, then wiped with a water-rinsed flannel and dried with a paper towel. A separate flannel is used for each axilla of each panellist. Each panellist wears a different product in each axilla and this is kept constant throughout the test.

A standard product application technique is used; for aerosols, a timed 2 second spray, 6 inches from the axilla is used; for pump sprays, a constant number of pumps is used. For roll-ons and sticks, product can either be applied as a fixed number of strokes or a known weight of product may be applied using a balance to measure the loss in weight of the product.

After 5 hours and 24 hours wear of the test product the odour intensity of each axilla is evaluated by each assessor. Each in turn assigns a score on the scale of 0 to 5, corresponding to the strength of the under-arm malodour.

After a 5 hour assessment panellists go about their normal business for the rest of the day. After 24 hours of the test, panellists are first evaluated for under-arm odour; then they are washed and the products re-applied. The protocol is repeated over 4 consecutive days. On the last day of the test panellists are assessed but no washing is carried out and no further product is applied.

Malodour scores for 5 and 24 hour wear periods are treated separately. Scores from the 4 day's assessments for all expert odour assessors are averaged to give a result for each test product for the week, and these are then averaged to give a team score. The results are analysed using a SAS-based Analysis of Variance routine which takes into account the factors which lead to variability, e.g. subject, day, left/right bias. The analysis also gives the difference in malodour score needed for a result to be statistically significant at the p=0.05 level. The Deodorant Value accorded to a test composition is the difference in score between the test composition and a placebo.

This test for determining the Deodorant Value of a deodorant composition for use in accordance with the invention is generally based on the test devised by Whitehouse and Carter as published in 'The Proceedings of the Scientific Section of the Toilet Goods Association", No. 48, December 1967 at pages 31-37 under the title "Evaluation of Deodorant Toilet Bars".

The test described in that publication has however generally been modified in three main ways: firstly, the product is applied directly to the axillae rather than by way of a soap bar wash, secondly, a 0 to 5 instead of a 0 to 10 grading scale was employed and, thirdly, grading of odour intensity was performed 5 hours and 24 hours instead of just 24 hours after treatment. This test is referred to herein as the Deodorant Value Test.

Although the invention in its widest aspect provides deodorant products comprising perfume compositions having an deodorant value of from 0.25 to 3.5, preferred deodorant skin treatment products are those comprising perfume compositions which having a deodorant value of at least 0.50, 0.60, 0.70, or 0.80, or 0.90, or 1.00, or even at least 1.20, the higher the minimum value, the more effective in the product as a deodorant product.

Deodorant perfume components can be classified into six chemically defined classes. The perfume components may be described in terms of four categories, each of which is given below together with examples of components which are to be assigned to each category.

1. Single chemical compounds whether natural or synthetic, for example, hexyl salicylate: the majority of components are in this category.

2. Synthetic reaction products (products of reaction), mixtures of isomers and possibly homologues, for example, alpha-iso-methyl ionone.

3. Natural oils and extracts, for example, benzoin Siam resinoid.

4. Synthetic oils (e.g. analogues of category 3): this category includes materials that are not strict analogues of natural oils but are materials that result from attempts to copy or improve upon certain natural oils, for example Bergamot AB 430.

Components of Categories (3) and (4) although often uncharacterised chemically are available commercially.

Where a material is supplied or used conventionally for convenience as a mixture, e.g. p-t-amyl cyclohexanone diluted with diethyl phthalate, for the purposes of this specification two components are present, so that use of 5% of a blend of 1 part of this ketone and 9 parts of diethylphthalate is represented as 0.5% of the ketone and 4.5% of diethyl phthalate.

It has been found advantageous in formulating the most effective deodorant perfumes for use in compositions according to the invention to use components that, as well as satisfying the lipoxidase or morpholine tests, satisfy further conditions. These conditions are:

i) there are at least five different components present conforming with the classification below;

ii) there are represented components from at least four different chemical classes (defined below):

iii) at least 50%, preferably at least 55% and most preferably , from 60 to 100% by weight of the deodorant perfumes comprise deodorant perfume components conforming with the classification below.

Each component should be allocated to one of six classes. These classes are:

| | |
|---|---|
| Class 1 - | Phenolic substances; |
| 2 - | Essential oils, extracts, resins and synthetic oils (denoted "AB"); |
| 3 - | Aldehyde and ketones; |
| 4 - | Nitrogen-containing compounds; |
| 5 - | Esters; |
| 6 - | Alcohols and ethers. |

In assigning a component to a class, the following rules are to be observed. Where the component could be assigned to more than one class, the component is allocated to the class occurring first in the order given above; for example methyl anthranilate, which is a nitrogen-containing compound, is placed in Class 4, although as an ester it otherwise might have been allocated to Class 5. Similarly, ethyl salicylate, which is phenolic in character, is allocated to Class 1 instead of Class 5.

The following are examples of deodorant perfume components that have either a Lipoxidase Inhibiting Capacity (LIC value) of at least 50% or a Raoult Variance Ratio (RVR value) of at least 1.1. Their class, molecular weight (m), LIC and RVR values as determined by the tests described herein are also indicated.

The nomenclature adopted for the components listed below and for the perfume ingredients which appear in the perfume formulations of Examples 1 to 7 is, so far as is possible, that employed by Steffen Arctander in "Perfume and Flavour Chemicals (Aroma Chemicals)" Volume 1 and II (1969) and the "Perfume and Flavour Chemicals (Aroma Chemicals)" Volume I and II (1969) and the "Perfume & Flavour Materials of Natural Origin" (1960) by the same author. Where a component or ingredient is not described by Arctander, then either the chemical name is given or, where this is not known, the perfumery house speciality code name is given. Note that synthetic oils denoted "AB" are available from Quest International Limited.

Specific examples of perfume components are:

| | LIC Value | RVR Value | m |
|---|---|---|---|
| **Class 1 - Phenolic Substances** | | | |
| iso-Amyl salicylate | 95 | 1.24 | 208 |
| Carvacrol | 32 | 1.43 | 150 |
| Clove leaf oil | 79 | 1.43 | 164 |
| Ethyl salicylate | - | 1.19 | 194 |
| iso-Eugenol | 100 | 1.48 | 164 |
| Hexyl salicylate | 100 | - | 222 |
| Thyme oil red | 55 | 1.37 | 150 |
| **Class 2 - Essential oils, extracts, resins and synthetic oils (denoted "AB")** | | | |
| Bergamot AB 430 | 58 | 0.97 | 175 |
| Geranium AB 76 | 26 | 1.29 | 154 |
| Rose AB 380 | 0 | 1.28 | 175 |
| Rose AB 409 | 35 | 1.34 | 175 |
| **Class 3 - Aldehydes and ketones** | | | |
| 7-Acetyl-1,1,3,4,4,6-hexamethyl-tetrahydronaphthalene | 100 | 1.03 | 258 |
| p-t-Amyl cyclohexanone | 50 | 1.10 | 182 |
| 2-n-Heptylcyclo-pentanone | 56 | 1.05 | 182 |
| α-iso-Methylionone | 100 | 1.13 | 206 |
| β-Methyl naphthyl ketone | 100 | 0.96 | 170 |

| Class 4 - Nitrogen-containing compounds | | | |
|---|---|---|---|
| | LIC value | RVR value | m |
| iso-Butyl quinoline | - | 1.10 | 185 |
| Methyl anthranilate | 69 | 1.20 | 151 |
| Class 5 - Esters | | | |
| o-t-Butylcyclohexyl acetate | 52 | 1.08 | 198 |
| Diethyl phthalate | 79 | 1.20 | 222 |
| Nonanediol-1,3-diacetate | 33 | 1.17 | 244 |
| Nonanolide-1,4 | 92 | 0.87 | 156 |
| i-Nonyl acetate | 50 | 0.83 | 186 |
| i-Nonyl formate | 19 | 1.49 | 172 |
| Phenylethyl phenyl acetate | 0 | 1.22 | 241 |
| Class 6 - Alcohols & Ethers | | | |
| Cinnamic alcohol | - | 1.28 | 134 |
| Dimyrcetol | 16 | 1.22 | 156 |
| 1,3,4,6,7,8-Hexahydro-4,6,6,7,8,8-hexamethyl cyclopenta-δ-2-benzopyran (Galaxolide) | 100 | - | 240 |
| Hydroxymethyl isopropylcyclopentane | 60 | 1.23 | 142 |
| 3a-Methyl-dodecahydro-6,6,9a-trimethylnaphtho-2(2,1-b)furan | 58 | 1.30 | 230 |
| Tetrahydromuguol | 24 | 1.23 | 158 |

Examples of other perfume ingredients are as follows:

| | LIC value | RVR value | m |
|---|---|---|---|
| Benzoin Siam resinoids | 87 | - | - |
| Benzyl salicylate | 0 | 1.58 | 228 |
| Bergamot AB 37 | 58 | 0.97 | 175 |
| p-t-Butyl cyclohexyl acetate | 54 | 0.98 | 198 |
| p-t-Butyl-α-methyl hydrocinnamic aldehyde (Lilial) | 74 | - | 204 |
| Coumarin | 58 | 1.22 | 146 |
| Ethyl vanillin | 100 | 1.43 | 152 |
| Geranium oil Bourbon | 26 | 1.29 | 154 |
| LRG 201 (a well known resorcyclic acid ester) | 100 | 1.21 | 196 |
| Mousse de chene Yugo | 98 | 1.29 | 182 |
| β-Naphthyl methyl ether | 100 | - | 158 |
| Opoponax resinoid | 96 | 1.33 | 150 |
| Patchouli oil | 76 | 1.25 | 140 |
| Petitgrain oil | 34 | 1.27 | 175 |
| Phenylethyl alcohol | 22 | 1.24 | 122 |
| Pimento leaf oil | 100 | - | 165 |
| Pomeransol AB 314 | 100 | - | - |

Of the components and ingredients listed herein, those that are preferred for their relatively milder odour intensity are (a) the ingredients included in the examples given below, and (b)

Bergamot AB,
Hexyl salicylate,
Rose AB 380
Diethyl phthalate,
Nonanediol 1,3-diacetate,
Isononyl acetate,
Cinnamic alcohol,
Benzoin Siam resinoid,
Benzyl salicylate,
p-t-butyl-alpha-methyl-hydrocinnamic aldehyde,
Opoponax resinoid.

A deodorant perfume should contain at least five different components, and from preferably at least four of the classes, preferably five or six. It is however possible, and indeed is usually advantageous, to employ more than five different components when formulating the perfume. Ideally, most if not all of the perfume is formulated from deodorant perfume components.

Components present in the deodorant perfume for purposes other than obtaining a deodorant effect, for example an adjunct like an anti-oxidant, may be excluded from the operation of the preceding instructions to the extent that the component is required for that other purpose. The levels at which adjuncts are conventionally present in perfumes or in perfumes or in products to which perfumes are added is well-established for conventional materials and readily determinable for new materials so that the application of the above exclusion presents no difficulty.

Odour Intensity Index Method

The samples are assessed by a panel of a suitable number, e.g. about 34, of assessors who have been trained to score the intensity of a sample using the magnitude estimation technique. This is a ratio scaling method in which the relative intensity of each sample is scored in ratio to the intensities of a range of odour standards (here, benzyl acetate diluted in dipropylene glycol at various concentrations.

1.5 g (+/- 0.1 g) of perfume, or 1.5 g (+/- 0.1 g) of benzyl acetate either neat or as a dilution in dipropylene

glycol, is placed into 7 ml white soda S.N.B. screw neck vials with 19 mm diameter necks. The samples are each coded and presented to the panel in a random order at least twice. A total of at least 64 assessments (or enough to reach statistical significance) is made for each sample by at least 16 panellists on each day over two days. Assessments are made in environmentally controlled assessment rooms using coloured lighting to ensure that panellists are not influenced by any slight colour differences between the samples.

Individual assessments are normalised and averaged to give a consensus intensity rating for each sample. The perceived intensities are expressed in arbitrary units and are derived from consensus magnitude estimates which are indicative of the ratio of perceived intensities, as follows:-

Each panellist was required to assess the intensity of a control sample (10% benzyl acetate solution in dipropylene glycol) in addition to each test fragrance and the reference samples. The intensity value (magnitude estimate) of the control sample was then used to normalise all the other assessments for each panellist as follows:-

$$\text{Normalised Intensity} = \frac{\text{Intensity of Unknown}}{\text{Intensity of control}} \times 100$$

or:

$$(I_N)_j = \frac{(i_k)_j}{(i_c)_j} \times 100$$

The normalised values for a sample were combined across all panellists to give a consensus value for the whole panel (the arithmetic mean).

$$\text{Odour intensity index} = \Sigma\ \frac{\text{(normalised panellist ratings)}}{\text{number of panellists}}$$

or:

$$I_K = \sum_{I}^{J} \frac{(I_N)_j}{J}$$

Where

| | |
|---|---|
| $I_k$ = | odour intensity index for sample k for the whole panel. |
| $(i_k)_i$ = | odour intensity of sample (magnitude estimate) as reported by j'th panellist. |
| $(I_N)_i$ = | single panellist's normalised datum. |
| n = | number of samples. |
| J = | number of panellists. |
| K = | sample number. |
| $(i_c)_j$ = | odour intensity of control (magnitude estimate) as reported by jth panellist. |

The invention is further illustrated by the following non-limitative examples.

Example 1

A fragrance composition which may be suitable for use in deodorant compositions along with a deodorant active material according to the invention is as follows;

| % | Component |
|---|---|
| 2.0 | Cedar wood oil (Virginian) |
| 2.0 | Cinnamic Alcohol |

(continued)

| % | Component |
|---|---|
| 13.0 | Diethyl Phthalate |
| 5.0 | Galaxolide DEP (50:50 mixture with diethyl phthalate) |
| 4.0 | Geranyl Phenylacetate |
| 1.0 | Guaiacwood oil (rectified) |
| 4.0 | Linalyl Benzoate |
| 6.0 | Moss Base AB 7004 (*) |
| 3.0 | Phenylethyl Phenylacetate |
| 20.0 | Rose Base AB 7003 (*) |
| 40.0 | Traseolide (Quest) |
| 100.0 | |

(*) (available from Quest International)

The odour type of this formulation is mildly flora, mossy, rose and musk. The composition is of low odour intensity and is suitable for incorporation into deodorant compositions, for example at a rate of incorporation of about 1.0% by weight of the deodorant composition, in conjunction with an effective amount of an inorganic deodorant active material.

Example 2

A further fragrance composition for use in deodorant compositions according to the invention is as follows:-

| % | Component |
|---|---|
| 8 | Benzyl Alcohol |
| 7.5 | Benzyl Salicylate |
| 2.0 | Cedar wool oil (Virginian) |
| 20.0 | Galaxolide DEP |
| 1.0 | Grisalva (10% solution in dipropylene glycol) (IFF) |
| 5.0 | Hercolyn D (Hercules) |
| 3.0 | Isobutyl Benzoate |
| 2.0 | Isobutyl Cinnamate |
| 1.0 | Linalyl Cinnamate |
| 5.0 | Moss Base AB 7004 (*) |
| 20.0 | Muguet Base AB 7001 (*) |
| 5.0 | Tonalid (7-acetyl-1,1,3,4,4,6-hexamethyl Itetrahydronaphthalene) (Polak's Frutal Works) |
| 20.0 | Traseolide (*) |
| 100.0 | |

The odour type of this formulation is mildly woody, mossy, muguet and musk.

Example 3

A further composition for use in deodorant compositions for extremely low odour intensity is as follows:

| % | Component |
|---|---|
| 5.0 | Benzyl Alcohol |
| 4.0 | Benzyl Cinnamate |
| 20.0 | Benzyl Salicylate |
| 1.0 | Cinnamyl Cinnamate |
| 5.0 | Diethyl Phthalate |
| 8.0 | Galaxolide DEP |

(continued)

| % | Component |
|---|---|
| 20.0 | Jasmin AB 7002 (*) |
| 5.0 | Linalyl Cinnamate |
| 2.0 | Sandalone AC 802 (*) |
| 30.0 | Traseolide (*) |
| 100.0 | |

The odour type of this formulation is mildly sweet, floral and musk.

Example 4

A further example of a perfumery composition for use in deodorant compositions according to the invention is as follows:-

| % | Component |
|---|---|
| 18.0 | Benzyl Salicylate |
| 2.0 | Diethyl Phthalate |
| 5.0 | Ethylene Brassylate |
| 10.0 | Galaxolide DEP |
| 20.0 | Muguet AB 7001 (*) |
| 2.0 | Phenylethyl Salicylate |
| 2.0 | Sandalone AC 802 (*) |
| 3.0 | Sandela (Givaudan) |
| 38.0 | Traseolide (*) |
| 100.0 | |

The odour type of this formulation is mildly muguet and musk.

Example 5

A further and preferred example of a perfumery composition for use in deodorant compositions according to the invention is as follows:-

| % | Component |
|---|---|
| 5.0 | Benzyl Alcohol |
| 8.0 | Benzyl Benzoate |
| 5.0 | Benzyl Cinnamate |
| 5.0 | Carnation AB 7005 (*) |
| 2.0 | Copaida Balsam |
| 10.0 | Galaxolide DEP |
| 15.0 | Hexyl Salicylate |
| 15.0 | Jasmin Base AB 7002 (*) |
| 35.0 | Traseolide (*) |
| 100.0 | |

Odour Intensity Indices

The odour intensity indices of the above-cited examples are as follows:

| Composition | Odour Intensity Index |
|---|---|
| Example 1 | 87 |

(continued)

| Composition | Odour Intensity Index |
|---|---|
| Example 2 | 85 |
| Example 3 | 72 |
| Example 4 | 79 |
| Example 5 | 75 |

For comparison and calibration, standard preparations of benzyl acetate have odour intensity indices as follows:-

| Reference Standard | Perceived Odour Index |
|---|---|
| 1% Benzyl Acetate | 54 |
| 5% Benzyl Acetate | 89 |
| 10% Benzyl Acetate | 102 |
| 20% Benzyl Acetate | 108 |
| 50% Benzyl Acetate | 120 |
| Neat Benzyl Acetate | 132 |
| (The standard samples of benzyl acetate are presented as dilutions in dipropylene glycol). | |

According to another preferred aspect of the invention, the composition of fragrance materials used in deodorant compositions according to the invention comprises at least 50% by weight of materials from at least four of the five categories of materials set out below:

a) at least 0.2%, preferably at least 0.5% and generally not more than 20% by weight of the perfume composition of one or more ethers of general formula

$$R^1OR^2$$

in which the groups $R^1$ and $R^2$ are connected only through the ether oxygen atom, and are aliphatic or aromatic groups such that the ether has a molecular weight of 150 to 200;

b) at least 2%, preferably at least 5%, and generally not more than 50% by weight of the perfume composition of one or more aromatic methyl ketones of general formula

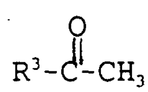

in which $R^3$ is an aromatic group such that the molecular weight of the ketone is from 170 to 300;

c) at least 2%, preferably at least 5% and generally not more than 50% by weight of the perfume composition of one or more alcohols of general formula

$$R^4OH$$

in which $R^4$ is an aliphatic group, optionally containing not more than one olefinic double bond, and optionally bearing an aromatic substituent group, such that the molecular weight of the alcohol is in the range 130 to 180;

d) at least 2% and generally not more than 40% by weight of the perfume composition of one or more acetates or propionates of general formula

$$CH_3CO_2R^5$$

and

$$C_2H_5O_2R^5$$

in which the group $R^5$ is an aliphatic group optionally containing not more than one olefinic double bond, and optionally bearing an aromatic substituent group such that the molecular weight of the ester is in the range 180 to 210;

e) at least 2% and generally not more than 60% by weight of the perfume composition of one or more salicylates of general formula

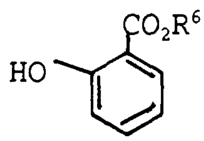

in which $R^6$ is an aliphatic group, optionally containing not more than one olefinic double bond, and optionally bearing an aromatic substituent group, such that the molecular weight of the salicylate is in the range 190 to 230; all the percentages being by weight of the whole perfume composition,

in conjunction with an inorganic deodorant active material, to form the deodorant composition suitable for topical application.

In one preferred aspect of this, the invention provides a perfume composition as defined above, in which the minimum of four categories which are required to be present include

i) both of categories (a) and (b) in which case category (a) contains from 0.2 to 6% preferably 0.5 to 6% by weight of one or more ethers in which the group $R^1$ is phenyl or naphthyl, optionally substituted with alkyl; and/or

ii) both of categories (a) and (e).

In another aspect this invention provides the use, in a deodorant composition, in conjunction with an inorganic deodorant active material, of a composition of fragrance materials as herein defined.

Preferably the perfume composition used in the deodorant composition includes category (a) as well as at least three others of the categories (b) to (e). More preferably, the perfume composition is in accordance with the preference above, so that the categories present include (a) and (e) and/or (b) with category (a) then containing from 0.2 to 6% preferably 0.5 to 6% by weight of the perfume composition of one or more ethers, in which the group $R^1$ is phenyl or naphthyl, optionally substituted with alkyl.

The five categories will now be reviewed in turn.

Category (a) - Ethers

These ethers are non-cyclic, in the sense that the ether oxygen atom is not part of a ring, although the groups $R^1$ and $R^2$ in the formula $R^1OR^2$ given above may themselves incorporate rings. Each of these groups may be aliphatic or aromatic e.g. alkyl, cycloalkyl, alkenyl, cycloalkenyl, phenyl, naphthyl, aryl substituted aliphatic or alkyl substituted aromatic. Preferably neither group contains more than one olefinic double bond.

The molecular weight range approximately corresponds to ethers containing up to about 13 or 14 carbon atoms in all. There will usually be at least 9 carbon atoms, depending however on any side chains present. Examples of ethers in this category are:-

Phenylethyl isoamyl ether, available under the trademark "ANTHER";

Phenylethyl n-butyl ether;
Benzyl isoamyl ether;
Dihydroanethole, which is 4-propylanisole, more properly known as methyl 4-propylphenyl ether;
Diphenyl oxide;
p-tert butylphenyl methyl ether, available under the trademark "EQUINOL";
Ethyl naphthyl ether, also known under the trademark "NEROLIN";
Methyl naphthyl ether, also known under the trademark "YARA".

The last five of the above ethers have at least one aromatic group which is phenyl, naphthyl or substituted phenyl or naphthyl.

Many of the ethers within category (a) are effective when used in rather small amounts. Generally if more than one ether is present, each ether will be present in an amount of at least 0.5% by weight of the perfume composition. It will generally be desirable that the total amount of these ethers does not exceed 20% by weight of the perfume composition and possibly does not exceed 10% if a mixed aliphatic aromatic ether is present. At least 1% is preferred. A quantity of not over 6% is preferred, for methyl naphthyl ether and/or ethyl naphthyl ether. The total of all ethers in category (a) may well not exceed 6%.

Category (b) - Aromatic Methyl Ketones

The group $R^3$ in the formula

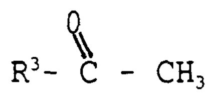

given above can contain up to approximately 18 carbon atoms and will usually contain at least 9. Examples of suitable ketones are:-

Alpha or beta methyl naphthyl ketone;
Musk ketone, which is a trivial name for 4-tert-butyl-3, 5-dinitro-2,6-dimethyl acetophenone;
1,1,2,4,47-hexamethyl-6-acetyl-1,2,3,4-tetrahydronaphthalene, available under the trademark "TONALID";
5-Acetyl-1,1,2,3,3,6-hexamethylindane, available under the trademark "PHANTOLIDE";
4-Acetyl-6-tert-butyl-1,1-dimethylindane, available under the trademark "CELESTOLIDE",
6-Acetyl-1-isopropyl-2,3,3,5-tetramethylindane, available under the trademark "TRASEOLIDE";
1,1,4,4-Tetramethyl-6-acetyl-7-ethyl-1,2,3,4-tetrahydronaphthalene, available under the trademark "VERSALIDE".

The amount of each ketone, if more than one is present, will desirably be at least 1% or at least 2% by weight of the perfume composition. The total amount of these ketones may extend up to 35% or even beyond, up to 50% by weight of the perfume composition. Possibly, however, the amount does not exceed 25%, 20% or 18% by weight of the perfume composition and may lie in a range from 5 to 15%. The amount may well be at least 5% or at least 10% by weight.

Category (c) - Alcohols

The group $R^4$ in the formula $R^4OH$ given above is aliphatic but may have an aromatic substituent. Olefinic unsaturation may be present to the extent of one double bond, but may be entirely absent. Aliphatic groups are therefore alkyl, alkenyl, cycloalkyl and cycloalkenyl, optionally bearing an aromatic substituent group.

The stated molecular weight range of 130 to 180 permits up to 11 carbon atoms in the group $R^4$. Usually there will be at least 8. Examples of suitable alcohols are:-

Cinnamic alcohol
Citronellol
Decanol
Dihydromyrcenol
Dimethylheptanol
Dimethyloctanol

Dimethyl benzyl carbinol
Isononanol
Isoborneol
4-isopropyl cyclohexanol
Isopulegol
Menthol
Myrtenol
Nonanol
Octanol
para-menthan-7-ol
2-tert-butylcyclohexanol
4-tert-butylcyclohexanol
3-methyl-5-phenyl pentanol, available under the trademark "PHENOXANOL"
2-Phenylpropanol
3-Phenylpropanol
9-Decen-1-ol, available under the trademark "ROSALVA" alpha-Terpineol
beta-Terpineol
Tetrahydrogeraniol
Tetrahydrolinalol
3,5,5-Trimethylcyclohexanol
Undecanol
10-Undecen-1-ol.

The amount of individual alcohols is preferably at least 1% or at least 2% by weight of the perfume composition. The total amount of alcohol is more preferably at least 5% but will generally not exceed 50% by weight of the perfume composition. From 8% to 40%, especially 8% to 30% or even 8% to 20%, is preferred.

Category (d) - Esters

These esters are acetates and propionates. Like the group $R^4$, discussed above, the group $R^5$ in the formula $CH_3CO_2R^5$ and $C_2H_5CO_2R^5$ given above is aliphatic, possibly with an aromatic substituent, and with no more than one olefinic double bond, if any.

The molecular weight range permits propionates in which $R^5$ has up to 9 carbon atoms, and acetates in which $R^5$ has up to 10 carbon atoms.

Examples of suitable esters are:-

3a,4,5,6,7,7a-hexahydro-4,7-methano-1(3)H-inden-6-yl propanoate, available under the trademark "FLOROCYCLENE";
3-acetoxymethyl-4,7,7-trimethylbicyclo[4.1.0]-hept-2-ene, available under the trademark "FORESTONE";
3a,4,5,6,7,7a-hexahydro-4,7-methano-1(3)H-inden-6-yl acetate, available under the trademark "JASMACYCLENE";
Bornyl acetate
Cinnamyl propionate
Citronellyl acetate
Decyl acetate
Dihydroterpinyl acetate
Dimethyl benzyl carbinyl acetate
3,5,5-trimethylhexyl acetate, available as "Inonyl acetate"
Isobornyl acetate
Isopulegol acetate
Menthyl acetate
Myrtenyl acetate
Myrtenyl propionate
Nonyl acetate
Terpinyl acetate
Terpinyl propionate
2-tert-butylcyclohexyl acetate
4-tert-butylcyclohexyl acetate

Tetrahydrogeranyl acetate
Tetrahydrolinalyl acetate
10-Undecenyl acetate.

The amounts of individual esters preferably are at least 1% or at least 2%. The total amount of esters may well be quite low, but can range up to as much as 40% by weight of the perfume composition or more. 2% to 30% is preferred.

The amount may be at least 5% or even at least 10%.

Category (e) - salicvlate

In the formula

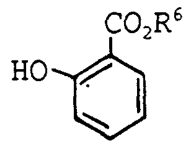

given above, the group $R^6$, like the groups $R^4$ and $R^5$ mentioned above, is aliphatic, possibly with an aromatic substituent, and either without olefinic unsaturation, or with one double bond at most. The requirement as to molecular weight permits groups $R^6$ of up to 11 carbon atoms. Examples of suitable salicylate are:-

Amyl salicylate
Benzyl salicylate
cis-3-hexenyl salicylate
Cyclohexyl salicylate
Hexyl salicylate
Isoamyl salicylate
Isobutyl salicylate

Salicylate can be used in large amounts, such as up to 50 or 60% by weight of the composition. At least 5%, better at least 10% or 15% may be preferred, and often at least 20% or even 25% will be preferred, e.g. 20% to 50% or 20% to 60%.

A material may have a structure such that it can be placed in more than one of the above categories. If so, the material should be placed only in a single category.

Preferably, however, the assignment of materials to categories is carried out in such a way that any material which is simultaneously more than one of either ester, alcohol, ketone or ether is first classified as an ester, alcohol, ketone or ether in that order of priority and then either attributed to the appropriate category (d), (c), (b) or (a) if the material satisfies the requirements for the category, or else excluded from all categories.

The effect of this approach is that Category (c) shall not then include any material which is an ester (regardless of whether it is an acetate, propionate or some other ester). Category (b) shall not include any material which is an ester or contains a hydroxyl group. Category (a) shall not include any material which is an ester, or contains a hydroxyl or keto group.

For example on this basis a material which was both an ether and an alcohol would be treated as an alcohol and placed in Category (c) if it satisfied the definition of category (c), or else excluded entirely. Similarly an ester which was not of formula:

$$CH_3CO_2R^5$$

or

$$C_2H_5CO_2R^5$$

would not be placed in any category.

As a practical matter, available salicylates do not have other functionality. However, it should be the case that categories (a) to (d) do not include any material which is a salicylate.

Further Materials

The fragrance compositions used in deodorant compositions of this invention may include other materials in addition to those in the above categories. These may include at least 2% by weight of the perfume composition falling within a sixth category of specified materials which are not all structurally related. Members of this further category (f) are:-

1. Aldehydes of formula $R^7CHO$ having molecular weight 180-220 in which $R^7$ is aliphatic or aryl-aliphatic, like $R^4$ and $R^5$. Especially envisaged are hexyl cinnamic aldehyde, and 2-methyl-3(para-t-butylphenyl) propionaldehyde which is available under the trademark "LILIAL".

2. 1,3,4,6,7,8-hexahydro-4,6,6,7,8,8-hexamethylcyclopenta-2-benzopyran, available under the trademark "GALAXOLIDE".

It may be the case that the Categories (a) to (d) or (a) to (e) do not include any material which contains an aldehyde group.

In addition to the preference for two plus two more out of five categories, additional groupings of categories may be advantageous. It is preferred that category (b) is present as well as categories (a) and (e).

The amount in category (e) may be at least equal to the amount in category (b). A preferred minimum of 10% of category (e) is equal to the minimum of categories (b) and (c) together if both are present.

It may also be preferred that five Categories are present out of the six Categories (a) to (f). Indeed it is preferred that all of categories (a), (b), (c), (d) and (e) are present.

The total amounts in categories (a)to (e) or (a) to (f) may be quite high, such as at least 65% or even at least 80% by weight of the perfume composition.

As already mentioned, the amount of each ether in category (a) may be at least 0.5% of the perfume composition. Additionally or alternatively, it may be the case that individual materials in at least three of categories (b), (c), (d) and (e) are present in amounts of at least 1% by weight of the perfume composition, and any materials from these categories present in amounts less than 1% by weight can be ignored when determining compliance with the requirements of this invention.

Some compositions exemplified in prior documents have included natural essential oils. Many such oils contain substantial amounts of terpenes and terpene aldehydes. These natural oils tend to give strong odours and preferably are not used in amounts greater than 25%, better not greater than 10% by weight of the composition.

Further information on such suitable fragrance compositions for use in deodorant compositions according to the invention may be found in EP 545,556 or EP 404,470, the contents of which are incorporated herein by reference.

The Lipoxidase Inhibition Test

In this test the capacity of a material to inhibit the oxidation of linoleic acid by lipoxidase (EC1.13.1.13) to form a hydroperoxide is measured.

Aqueous 0.2M sodium borate solution (pH 9.0) is used as buffer solution.

A control substrate solution is prepared by dissolving linoleic acid (2.0 ml) in absolute ethanol (60 ml), diluting with distilled water to 100 ml and then adding borate buffer (100 ml) and absolute ethanol (300 ml).

A test substrate solution is prepared in the same way as the control substrate solution except that for the absolute ethanol (300 ml) is substituted the same volume of a 0.5% by weight solution in ethanol of the material to be tested.

A solution of the enzyme lipoxidase in the borate buffer and having an activity within the range of from 15,000 to 40,000 units per ml is prepared.

The activity of the lipoxidase in catalysing the oxidation of linoleic acid is first assayed spectrophotometrically using the control. An automatic continuously recording spectrophotometer is used and the increase in extinction at 234 nm (the peak of hydroperoxide) is measured to follow the course of oxidation, the enzyme concentration used being such that it gives an increase in optical density ($\Delta$OD) at 234 nm within the range of from 0.6 to 1.0 units per minute. The following ingredients are placed in two 3 ml cuvettes:

| | Control (ml) | Blank (ml) |
|---|---|---|
| Control substrate solution | 0.10 | 0.10 |
| Absolute ethanol | 0.10 | 0.10 |
| Borate buffer | 2.75 | 2.80 |
| Lipoxidase solution | 0.05 | - |

The lipoxidase solution is added to the control cuvette last and the reaction immediately followed spectrophotometrically for about 3 minutes, with recording of the increase in optical density at 234 nm as a curve on a graph.

The capacity of a material to inhibit the oxidation is then measured using a test sample containing enzyme, substrate and a deodorant material. The following ingredients are placed in two 3 ml cuvettes.

| | Test Sample (ml) | Blank (ml) |
|---|---|---|
| Test substrate solution | 0.10 | 0.10 |
| Absolute ethanol | 0.10 | 0.10 |
| Borate buffer | 2.75 | 2.80 |
| Lipoxidase solution | 0.05 | - |

The lipoxidase solution is added to the test sample cuvette last and the course of the reaction immediately followed as before.

The lipoxidase-inhibiting capacity of the material is then calculated from the formula 100 $(S_1-S_2)/S_1$, where $S_1$ is the slope of the curve obtained with the control and $S_2$ is the slope of the curve obtained with the test sample, and thus expressed as % inhibitor. A material that gives at least 50% inhibition in the test is hereafter referred to as having a lipoxidase-inhibiting capacity of at least 50%.

The Morpholine Test

In this test the capacity of a material to depress the partial vapour pressure of morpholine more than that required by Raoult's Law is measured. Substances that undergo chemical reaction with morpholine, for example aldehydes, are to be regarded as excluded from the test.

Into a sample bottle of capacity 20 ml is introduced morpholine (1 g), the bottle fitted with a serum cap and then maintained at 37°C for 30 minutes for equilibrium to be reached. The gas in the headspace of the bottle is analysed by piercing the serum cap with a capillary needle through which nitrogen at 37°C is passed to increase the pressure in the bottle by a standard amount and then allowing the excess pressure to inject a sample from the headspace into gas chromatograph apparatus, which analyses it and provides a chromatographic trace curve with a peak due to morpholine, the area under which is proportional to the amount of morpholine in the sample.

The procedure is repeated under exactly the same conditions using instead of morpholine alone, morpholine (0.25 g) and the material to be tested (1 g); and also using the material (1 g) without the morpholine to check whether it gives an interference with the morpholine peak (which is unusual).

The procedure is repeated until reproducible results are obtained. The areas under the morpholine peaks are measured and any necessary correction due to interference by the material is made.

A suitable apparatus for carrying out the above procedure is a Perkin-Elmer Automatic GC Multifract F40 for Head Space Analysis. Further details of this method are described by Kolb in "CZ-Chemie-Technik", Vol 1, No 2, 87-91 (1972) and by Jentzsch et al in "Z. Anal. Chem." 236, 96-118 (1968).

The measured areas representing the morpholine concentration are proportional to the partial vapour pressure of the morpholine in the bottle headspace. If A is the area under the morpholine peak when only morpholine is tested and A' is the area due to morpholine when a material is present, the relative lowering of partial vapour pressure of morpholine by the material is given by 1-A'/A.

According to Raoult's Law, if at a given temperature the partial vapour pressure or morpholine in equilibrium with air above liquid morpholine is p, the partial vapour pressure p' exerted by morpholine in a homogeneous liquid mixture of morpholine and material at the same temperature is pM/(M+PC), where M and PC are the molar concentrations of morpholine and material. Hence, according to Raoult's Law the relative lowering of morpholine partial vapour pressure (p-p')/p, is given by 1-M/(M+PC), which under the circumstances of the test is 87(87+m/4), where m is the molecular weight of the perfume material.

The extend to which the behaviour of the mixture departs from Raoult's Law is given by the ratio

$$\frac{1-A'/A}{87/(87+m/4)}$$

The above ratio, which will be referred to as the Raoult variance ratio, is calculated from the test results. Where a material is a mixture of compounds, a calculated or experimentally determined average molecular weight is used for m. A material that depresses the partial vapour pressure of morpholine by at least 10% more than that required by Raoult's Law in the Morpholine Test is one in which the Raoult variance ratio is at least 1.1.

A large number of materials which satisfy one or both tests are described in this specification, and these are hereafter referred to as "components", in contrast to other materials which fail both tests which are referred to as "ingredients".

Preferred fragrance compositions for use in deodorant compositions according to the invention have either a lipoxidase inhibiting capacity (LIC) of at least 50%, or have a Raoult variance ratio (RVR) of at least 1.1. Further examples of such components can be found in GB 2016507 (Unilever), the content of which is incorporated herein by reference.

In preferred fragrance compositions for use in deodorant compositions according to the invention, at least 40% by weight of the components in the fragrance composition have an LIC of at least 50%, or an RVR of at least 1.1. Preferably at least 40% by weight of the components in the fragrance composition have an RVR of at least 1.1.

Example 6

Compositions as described below containing a fragrance composition analagous with Example 5 above were tested in topical compositions for their Deodorant Value, according to the Deodorant Value Test protocol described above. All test compositions were dissolved or suspended in distilled water as the cosmetic vehicle, with an additional alcohol/water formulation (Composition D) acting as a positive control.

| | Composition (% w/w) | | | |
|---|---|---|---|---|
| Component | A | B | C | D |
| Fragrance Comp. | 1.5 | 1.5 | - | - |
| Zinc Phenol Sulphonate | 6.0 | - | 6.0 | - |
| Ethanol | - | - | - | 95 |

Compositions A-D were assessed after 5 and 24 hours for their odour scores as measured by the protocol described above under the heading "Deodorant Value Test" above. The results are shown below;

| | Odour Score | | | |
|---|---|---|---|---|
| Composition | 5 hours | 5 hour Deodorant Value* | 24 hours | 24 hour Deodorant Value* |
| A | 1.18 | 1.37 | 1.42 | 1.30 |
| B | 1.72 | 0.83 | 2.33 | 0.39 |
| C | 2.41 | 0.14 | 2.23 | 0.49 |
| D | 2.29 | 0.26 | 2.27 | 0.45 |

* As calculated against an average placebo value; see below.

The figures for odour reduction from this test can also be represented as a percentage odour reduction value, against an average placebo value obtained from a number of previous tests using the same protocol on the same panel of subjects. These tests have shown the average placebo value for the 5 hour reading to be 2.55, and for the 24 hour reading to be 2.72.

The odour reduction values from this test can therefore be recalculated as a % odour reduction against the average placebo value as follows;

$$\%\ \text{odour reduction} = \frac{\text{placebo - test value}}{\text{placebo value}} \times 100$$

This calculation gives rise to the following % odour reduction scores.

| | % Odour Score | |
|---|---|---|
| Composition | 5 hours | 24 hours |
| A | 54 | 48 |
| B | 33 | 14 |
| C | 6 | 18 |

As can clearly be seen, even for the more demanding 24 hour test the % odour reduction from composition A, which contains both the fragrance composition and the zinc salt, is notably greater than the sum of the % odour reduction values for compositions B and C, which contain the perfume composition and zinc salt respectively.

## Claims

**1.** A deodorant composition suitable for topical application to the human skin, comprising a composition of:

non-encapsulated fragrance materials which have an Odour Intensity Index of less than about 110, when tested according to an Odour Intensity Test as described herein, and a Deodorant Value greater than 0.25, as measured by the Deodorant Value Test, and
a deodorant active metal salt or complex in a cosmetically suitable vehicle.

**2.** A deodorant composition according to claim 1 wherein the deodorant metal salt or complex is a zinc salt or complex.

**3.** A deodorant composition according to claim 2 wherein the zinc salt in zinc phenolsulphonate.

**4.** A deodorant composition according to claim 1, wherein the deodorant metal salt or complex is an aluminium or zirconium containing deodorant active salt or complex.

**5.** A deodorant composition according to any of the preceding claims, wherein the deodorant metal salt or complex is present at a level of 0.1-12% by weight of the composition.

**6.** A deodorant composition according to any of claims 1-5, wherein the composition of fragrance materials comprises at least 50% by weight of materials from at least four of the five categories of materials set out below:

a) at least 0.2%, and generally not more than 20% by weight of the perfume composition of one or more ethers of general formula

$$R^1OR^2$$

in which the groups $R^1$ and $R^2$ are connected only through the ether oxygen atom, and are aliphatic or aromatic groups such that the ether has a molecular weight of 150 to 200;

b) at least 2%, and generally not more than 50% by weight of the perfume composition of one or more aromatic methyl ketones of general formula

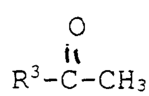

in which $R^3$ is an aromatic group such that the molecular weight of the ketone is from 170 to 300;

c) at least 2%, and generally not more than 50% by weight of the perfume composition of one or more alcohols of general formula

$$R^4OH$$

in which $R^4$ is an aliphatic group, optionally containing not more than one olefinic double bond, and optionally bearing an aromatic substituent group, such that the molecular weight of the alcohol is in the range 130 to 180;

d) at least 2% and generally not more than 40% by weight of the perfume composition of one or more acetates or propionates of general formula

$$CH_3CO_2R^5$$

and

$$C_2H_5O_2R^5$$

in which the group $R^5$ is an aliphatic group optionally containing not more than one olefinic double bond, and optionally bearing an aromatic substituent group such that the molecular weight of the ester is in the range 180 to 210;

e) at least 2% and generally not more than 60% by weight of the perfume composition of one or more salicylates of general formula

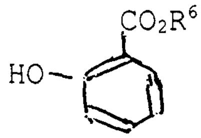

in which $R^6$ is an aliphatic' group, optionally containing not more than one olefinic double bond, and optionally bearing an aromatic substituent group, such that the molecular weight of the salicylate is in the range 190 to 230; all the percentages being by weight of the whole perfume composition.

**Patentansprüche**

1. Deodorantzusammensetzung, geeignet zur örtlichen Anwendung auf die menschliche Haut, umfassend eine Zusammensetzung von: nicht eingekapselten Duftstoffmaterialien, die einen Odour-Intensity-Index von weniger als etwa 110 aufweisen, wenn sie gemäß einem hierin beschriebenen Odour-Intensity-Test getestet werden, und einen Deodorant-Value größer als 0,25 aufweisen, wenn sie mit dem Deodorant-Value-Test gemessen werden und ein/en Deodorantaktivstoffmetallsalz oder -komplex in einem kosmetisch geeigneten Träger.

2. Deodorantzusammensetzung nach Anspruch 1, wobei das/der Deodorantmetallsalz oder -komplex ein Zinksalz oder -komplex ist.

3. Deodorantzusammensetzung nach Anspruch 2, wobei das Zinksalz Zinkphenolsulfonat ist.

4. Deodorantzusammensetzung nach Anspruch 1, wobei das/der Deodorantmetallsalz oder -komplex ein Aluminium oder Zirconium enthaltendes/r Deodorantaktivstoffsalz oder -komplex ist.

5. Deodorantzusammensetzung nach einem der vorangehenden Ansprüche, wobei das/der Deodorantmetallsalz oder -komplex zu einem Anteil von 0,1-12 Gewichtsprozent der Zusammensetzung vorliegt.

6. Deodorantzusammensetzung nach einem der Ansprüche 1-5, wobei die Zusammensetzung von Duftstoffmaterialien mindestens 50 Gewichtsprozent Materialien von mindestens vier der nachstehend angeführten fünf Kategorien von Materialien umfasst:

a) mindestens 0,2% und im Allgemeinen nicht mehr als 20 Gewichtsprozent der Parfümzusammensetzung von einem oder mehreren Ethern der allgemeinen Formel

$$R^1OR^2,$$

worin die Gruppen $R^1$ und $R^2$ nur durch das Ethersauerstoffatom verbunden sind und aliphatische oder aromatische Gruppen darstellen, so dass der Ether ein Molekulargewicht von 150 bis 200 aufweist;
b) mindestens 2% und im Allgemeinen nicht mehr als 50 Gewichtsprozent der Parfümzusammensetzung von einem oder mehreren aromatischen Methylketonen der allgemeinen Formel

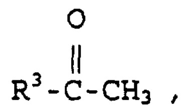

worin $R^3$ eine aromatische Gruppe darstellt, so dass das Molekulargewicht des Ketons 170 bis 300 ist;
c) mindestens 2% und im Allgemeinen nicht mehr als 50 Gewichtsprozent der Parfümzusammensetzung von einem oder mehreren Alkoholen der allgemeinen Formel

$$R^4OH,$$

worin $R^4$ eine aliphatische Gruppe darstellt, die gegebenenfalls nicht mehr als eine olefinische Doppelbindung enthält und gegebenenfalls eine aromatische Substituentengruppe trägt, so dass das Molekulargewicht des Alkohols im Bereich 130 bis 180 liegt;
d) mindestens 2% und im Allgemeinen nicht mehr als 40 Gewichtsprozent der Parfümzusammensetzung von einem oder mehreren Acetaten oder Propionaten der allgemeinen Formel

$$CH_3CO_2R^5$$

und

$$C_2H_5O_2R^5,$$

worin die Gruppe $R^5$ eine aliphatische Gruppe darstellt, die gegebenenfalls nicht mehr als eine olefinische Doppelbindung enthält und gegebenenfalls eine aromatische Substituentengruppe trägt, so dass das Molekulargewicht des Esters im Bereich 180 bis 210 liegt;
e) mindestens 2% und im Allgemeinen nicht mehr als 60 Gewichtsprozent der Parfümzusammensetzung von einem oder mehreren Salicylaten der allgemeinen Formel

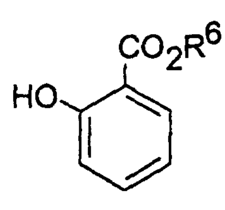

worin $R^6$ eine aliphatische Gruppe darstellt, die gegebenenfalls nicht mehr als eine olefinische Doppelbindung enthält und gegebenenfalls eine aromatische Substituentengruppe trägt, so dass das Molekulargewicht des Salicylats im Bereich 190 bis 230 liegt; wobei alle Prozentangaben auf das Gewicht der gesamten Parfümzusammensetzung bezogen sind.

**Revendications**

**1.** Composition désodorisante convenant à une application topique à la peau humaine, comprenant une composition de substances parfumantes non encapsulées qui ont un indice d'intensité d'odeur inférieur à environ 110, lorsqu'on les teste conformément à un test d'intensité d'odeur décrit ici, et une valeur désodorisante supérieure à 0,25, mesurée par le test de valeur désodorisante, et un sel ou un complexe de métal actif désodorisant dans un véhicule acceptable du point de vue cosmétique.

**2.** Composition désodorisante selon la revendication 1, le sel ou le complexe de métal désodorisant étant un sel ou un complexe de zinc.

**3.** Composition désodorisante selon la revendication 2, dans laquelle le sel de zinc est du phénolsulfonate de zinc.

**4.** Composition désodorisante selon la revendication 1, dans laquelle le sel ou le complexe de métal désodorisant est un sel ou un complexe actif désodorisant contenant de l'aluminium ou du zirconium.

**5.** Composition désodorisante selon l'une quelconque des revendications précédentes, dans laquelle le sel ou le complexe de métal désodorisant est présent à une teneur de 0,1 à 12% en poids de la composition.

**6.** Composition désodorisante selon l'une quelconque des revendications 1 à 5, dans laquelle la composition de substances parfumantes comprend au moins 50% en poids des substances appartenant à au moins quatre des cinq catégories de substances présentées ci-dessous :

a) au moins 0,2%, et généralement pas plus de 20% en poids de la composition de parfum, d'un ou plusieurs éthers de formule générale

$$R^1OR^2$$

dans laquelle les groupes $R^1$ et $R^2$ ne sont reliés que par l'atome d'oxygène de l'éther, et sont des groupes aliphatiques ou aromatiques tels que l'éther ait une masse moléculaire de 150 à 200 ;

b) au moins 2%, et généralement pas plus de 50% en poids de la composition de parfum, d'une ou plusieurs méthylcétones aromatiques de formule générale :

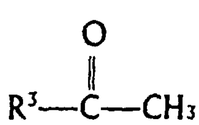

dans laquelle $R^3$ représente un groupe aromatique tel que la masse moléculaire de la cétone soit comprise entre 170 et 300 ;

c) au moins 2%, et généralement pas plus de 50% en poids de la composition de parfum, d'un ou plusieurs alcools de formule générale :

$$R^4OH$$

dans laquelle $R^4$ représente un groupe aliphatique, contenant éventuellement pas plus d'une double liaison oléfinique, et portant éventuellement un groupe substituant aromatique, de telle sorte que la masse moléculaire de l'alcool soit comprise dans l'intervalle allant de 130 à 180 ;

d) au moins 2%, et généralement pas plus de 40% en poids de la composition de parfum, d'un ou plusieurs acétates ou proprionates de formule générale :

$$CH_3CO_2R^5$$

et

$$C_2H_5O_2R^5$$

dans lesquelles le groupe $R^5$ représente un groupe aliphatique contenant éventuellement pas plus d'une double liaison oléfinique, et portant éventuellement un groupe substituant aromatique tel que la masse moléculaire de l'ester soit comprise dans l'intervalle allant de 180 à 210.

e) au moins 2%, et généralement pas plus de 60% en poids de la composition de parfum, d'un ou plusieurs des salicylates de formule générale :

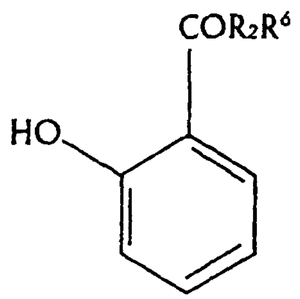

dans laquelle $R^6$ représente un groupe aliphatique, contenant éventuellement pas plus d'une double liaison oléfinique, et portant éventuellement un groupe substituant aromatique, tel que la masse moléculaire du salicylate soit comprise dans l'intervalle allant de 190 à 230 ; tous les pourcentages étant en poids de la composition de parfum entière.